# EUROPEAN PATENT APPLICATION

(11) **EP 4 417 608 A1**
(43) Date of publication of application: **21.08.2024**
(21) Application number: 22889437.4
(22) Date of filing: 04.11.2022
(51) Int. Cl.: C07D 487/04, A61K 31/53, A61P 31/12, A61P 31/14

(54) **CRYSTAL FORM OF ISOBUTYRATE NUCLEOSIDE COMPOUND, AND PREPARATION METHOD**

(30) Priority: 04.11.2021 CN 202111304134; 23.03.2022 CN 202210285185
(71) Applicant: Shenzhen Antiv Pharma Co., Ltd., Shenzhen, Guangdong 518000 (CN)
(72) Inventor: ZHANG, Xumu, Shenzhen, Guangdong 518000 (CN); LI, Yingjun, Shenzhen, Guangdong 518000 (CN); ZHOU, Qifan, Shenzhen, Guangdong 518000 (CN); LI, Guanguan, Shenzhen, Guangdong 518000 (CN)
(74) Representative: Herrmann, Uwe
(86) International application number: PCT/CN2022/129970
(87) International publication number: WO 2023/078416

(57) **Abstract**

The present invention provides a crystalline form of an isobutyrate nucleoside compound and a preparation method thereof, wherein the crystalline form is III; the X-ray powder diffraction pattern has characteristic peaks at 2θ values of 8.47 ± 0.2°, 16.87 ± 0.2°, 17.47 ± 0.2°, 22.13 ± 0.2° and 28.66 ± 0.2°. The crystalline form III has good physical and chemical stability. The preparation method of the crystalline form has advantages of simple operations, high repeatability.

## Description

### TECHNICAL FIELD

The present invention pertains to the field of medical technology, relates to crystal form of isobutyrate nucleoside compound, and preparation method.

### BACKGROUND

2019 novel coronavirus (Severe Acute Respiratory Syndrome Coronavirus 2, SARS-CoV-2) is an enveloped, single-stranded RNA virus, belonging to the genus β coronavirus. Similar to SARS and MERS, SARS-CoV-2 genome encodes non-structural proteins including 3-chymotrypsin-like protease (3CLpro), papain-like protease (PLpro), helicase and RNA-dependent RNA polymerase (RdRp), as well as structural proteins such as spike glycoproteins and accessory proteins. The surface spike protein of SARS-CoV-2 binds the angiotensin converting enzyme 2 (ACE2) receptor on the surface of human cells to infect human respiratory epithelial cells. After entering the host cell, the virus disintegrates and the nucleocapsid and viral RNA were released into the cytoplasm. The open reading frame at the 5'-terminal of viral RNA (ORF1a/b) encodes polyproteins (pp1a and pp 1 ab) playing an essential role in the processing and maturation of enzymes required for viral replication. PP1a and PP1ab can be cleaved by PLPro and 3CLPro to produce non-structural proteins, including helicase and RNA-dependent RNA polymerase *etc.,* they play a key role in the transcription and replication of SARS-CoV-2. Currently, these four proteins, including the surface spike glycoprotein of coronavirus recognition receptor, and 3CLpro, PLpro and RdRp those are important proteins involved in viral reproduction and transcription process, are very attractive targets for the development of antiviral drugs.

The compound of formula A is a nucleoside analogue acting on RdRp with good oral bioavailability and good anti-coronavirus activity in vitro and in vivo, and the applicant of the present application had filed patent applications describing the use of the compound for treating viral infection and the synthetic process, for details, see Chinese Patent Application Nos 202111083730.9 and 202110621245.6.

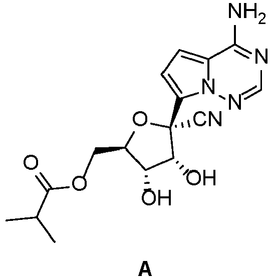

Drug polymorphism is a common phenomenon in drug research and an important factor affecting drug quality. Various crystalline forms of the same drug have significantly different appearance, solubility, melting point, dissolution, bioavailability, and so on, also have different effects on stability, bioavailability and efficacy and so on. Therefore, the polymorphism of a drug should be considered overall in drug research.

The inventors conducted relevant research on crystalline forms of the compound of formula A and found a crystalline form having advantages of simple preparation process, good stability, and high solubility.

### SUMMARY

The present invention primarily relates to crystalline form III of the compound of formula A. The partial crystalline forms provided herein have advantages of simple preparation process, high reproducibility contributing to industrial production, good physical and chemical stability suitable in formulations.

In other aspect, the present invention also provides a preparation process, a composition and use, *etc.* of crystalline form III of the compound of formula A.

In first aspect, the present invention provides a crystalline form of the compound of formula A.

Crystalline form III of the compound of formula A,

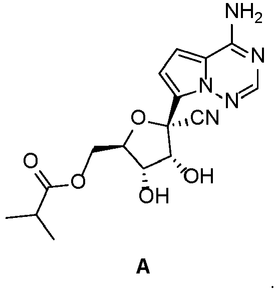

In some embodiments, the X-ray powder diffraction pattern of crystalline form III of the compound of formula A has characteristic peaks at 2θ values of 8.47 ± 0.2 °, 16.87 ± 0.2 °, 17.47 ± 0.2 °, 22.13 ± 0.2 ° and 28.66 ± 0.2 °.

In some embodiments, the X-ray powder diffraction pattern of crystalline form III of the compound of formula A has characteristic peaks at 2θ values of 8.47±0.2°, 16.87±0.2°, 17.47±0.2°, 22.13±0.2°, 28.66±0.2°, 10.52±0.2°, 11.80±0.2°, 14.04±0.2°.

In some embodiments, the X-ray powder diffraction pattern of crystalline form III of the compound of formula A has characteristic peaks at 2θ ± 0.2° values of 8.475, 10.523, 11.808, 14.043, 15.331, 16.878, 17.470, 20.293, 21.106, 22.131, 23.733, 24.074 , 24.545, 24.915, 25.688, 26.147, 26.528, 26.778, 27.526, 28.079, 28.669, 30.126, 30.783, 31.044, 31.689, 32.110, 32.793, 33.304, 34.868, 35.431 35.746, 36.494, 37.007, 37.887.

In some embodiments, the X-ray powder diffraction pattern of crystalline form III of the compound of formula A is substantially as depicted in Fig.1; the differential scanning calorimetry (DSC) curve of crystalline form III of the compound of formula A is substantially as depicted in Fig.4; the thermogravimetric analysis (TGA) curve is substantially as depicted in Fig. 5; In some embodiments, crystalline form III is essentially pure and crystalline form III is substantially free of one or more other crystalline forms.

In second aspect, the present invention provides a process for preparing crystalline form III of the compound of formula A described in the first aspect.

A process for preparing crystalline form III of the compound of formula A described in the first aspect, comprises:
dissolving the compound of formula A in water at room temperature, cooling to -5.0 °C to 5.0 °C, adding an esters solvent, precipitating solids, filtering, and evaporating the solvent in the filter cake to obtain the crystalline form.

For every 100 grams of the compound of formula A, the amount of water used can be 200 ml to 500 ml, and the amount of the esters solvent used can be 100 ml to 200 ml. In some embodiments, for every 1 gram of the compound of formula A, the amount of water used can be 50 to 300 ml, and the amount of the esters solvent used can be 50 to 200 ml. In some embodiments, the esters solvent is selected from methyl formate, ethyl acetate or isobutyl acetate, and so on.

In some embodiments, the process for preparing crystalline form III comprises: adding water, organic acid and INT-1 into a reaction vessel at the temperature of 20 °C to 30 °C; stirring for more than 24 hours at room temperature; sampling and testing the reaction endpoint (if the peak area of INT-2 is less than 0.5%, it is considered as the reaction has ended); cooling to -5.0 °C to 5.0 °C; adding an esters solvent; adding an alkaline aqueous solution to adjust pH value to 7-8; stirring for more than 1 hour; filtering; washing the filter cake with water.

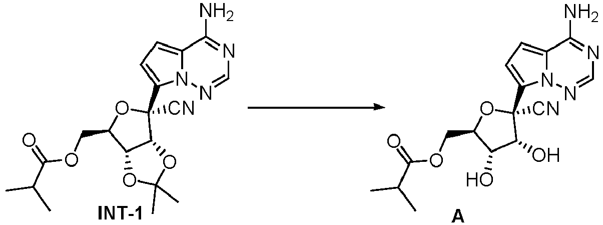

In some embodiments, the organic acid is formic acid or acetic acid, and the volume ratio of the organic acid to water is 3:1 to 1: 1; preferably about 2:1; the esters solvent is selected from methyl formate, ethyl acetate or isobutyl acetate, and so on.

In third aspect, the present invention provides a composition.

A composition comprises the crystalline form of the first aspect and a pharmaceutically acceptable adjuvant.

Calculated by mass ratio, the crystalline form is at least 0.05% -95% of the total mass of the composition.

In forth aspect, the present invention provides use of the aforementioned crystalline form or the aforementioned composition.

The composition comprises traditional Chinese medicine components and/or western medicine components. The western medicine components comprise Apilimod, R82913, DS-6930, ONO 5334, Remdesivir, Oseltamivir phosphate, Tetrandrine, Clofazimine, Astemizole, Recombinant Human Angiotensin Converting Enzyme 2 or Favipiravir and/or at least one pharmaceutically acceptable salt thereof.

The use of the crystalline form of the first aspect or the composition of the third aspect for the manufacture of a product for preventing, alleviating and/or treating a virus infection, or the replication or propagation thereof, or the cytopathic effects thereof.

The Use of the crystalline form of the first aspect or the composition of the third aspect for the manufacture of a product for preventing, alleviating and/or treating a coronavirus infection, or the replication or propagation of a homologous variant thereof, and the cytopathic effects thereof.

The coronavirus comprises MHV-A59, HCoV-229E, HCoV-OC43, HCoV-NL63, HCoV-HKU1, SARS-CoV, MERS-CoV, SARS-CoV-2, mouse hepatitis virus, Feline infectious peritonitis virus, canine coronavirus, bovine coronavirus, avian infectious bronchitis virus or porcine coronavirus.

### Beneficial Effects

Compared with prior art, the invention has the following technical effects: crystalline form III of the compound of formula A disclosed herein has good physical and chemical stability, which is conducive to storage and meets the requirement of drug stability, and it has good solubility and high bioavailability; the production process is simple and is beneficial to industrial production.

### Definitions

Unless otherwise indicated, the following terms and phrases as used herein are intended to have the following meanings

"The compound of the present invention" refers to the compound of formula A or a pharmaceutically acceptable salt, tautomer, polymorph, isomer or solvate thereof. Similarly, "the compound of formula A" refers to the compound or a pharmaceutically acceptable salt, tautomer, polymorph, isomer or solvate thereof.

In the present invention, "compound A" and "compound of formula A" represent the same compound.

The term "crystalline form" refers to a crystalline structure formed by different arrangement of molecules in lattice space due to intramolecular or intermolecular bonding.

The term "essentially pure" refers that a crystalline form is substantially free of one or more other crystalline forms, the purity is at least 60%, or at least 70%, or at least 80%, or at least 85%, or at least 90%, or at least 93%, or at least 95%, or at least 98%, or at least 99%, in addition to this main crystalline form, a small amount of other crystalline form can also be mixed in it. The weight percentage of other crystalline forms is less than 20%, or less than 10%, or less than 5%, or less than 3%, or less than 1%, or less than 0.5%, or less than 0.1%, or less than 0.01%.

The term "substantially free of one or more other crystalline forms" refers that the percentage of other crystalline forms in the total weight is less than 20%, or less than 10%, or less than 5%, or less than 3%, or less than 1%, or less than 0.5%, or less than 0.1%, or less than 0.01%.

The term "substantially as depicted in Fig." means at least 50%, or at least 60%, or at least 70%, or at least 80%, or at least 90%, or at least 95%, or at least 99% of the characteristic peaks exist in the X-ray powder diffraction pattern appear in the given X-ray powder diffraction pattern.

In the context of the present invention, regardless of whether words such as "approximately" or "about" are used, all numbers disclosed herein are approximate values. There may be differences of 1%, 2%, 5%, 7%, 8%, 10%, 15%, or 20% and so on in each number. Whenever a number having a value N is disclosed, any number having the value N+/-1%, N+/-2%, N+/-3%, N+/-5%, N+/-7%, N+/-8%, N+/-10%, N+/-15%, or N+/-20% will be explicitly disclosed, where "+/-" refers to plus or minus. Whenever a numerical range with a lower limit, RL, and an upper limit, RU, is disclosed, any number falling within the range is specifically disclosed. In particular, the following numbers within the range are specifically disclosed: R=RL+K*(RU-RL), wherein K is a variable ranging from 1% to 100% with a 1% increment. Such as 1%, 2%, 3%, 4%, 5%......50%, 51%, 52%......95%, 96%, 97%, 98%, 99%, or 100%. Moreover, any numerical range defined by two R numbers as defined above is also specifically disclosed.

In the context of the present invention, the values of 2θ in the X-ray powder diffraction pattern are all in degrees (°).

"V/V" represents a volume ratio. IC₅₀ represents half inhibitory concentration.

"wt%" represents a mass percentage of a component in the total mass of a mixture.

In the present invention, "room temperature" refers to the ambient temperature, which ranges from approximately 10 °C to approximately 40 °C. In some embodiments, "room temperature" refers to a temperature ranging from approximately 20 °C to approximately 30 °C; In other embodiments, "room temperature" refers to a temperature ranging from approximately 25 °C to approximately 30 °C; In some other embodiments, "room temperature" refers to 10 °C, 15 °C, 20 °C, 25 °C, 30 °C, 35 °C, 40 °C, etc.

The term "treating", as used herein, unless otherwise indicated, means reversing, alleviating the disorder or condition to which such term applies, or one or more symptoms of such disorder or condition, or inhibiting the progression of the disorder or condition or one or more symptoms of such disorder or condition, or preventing the disorder or condition or one or more symptoms of such disorder or condition. The term "treatment", as used herein, refers to the act of treating, as "treating" is defined immediately above.

### Instrument parameters

Unless otherwise specified in the parameter description, all analyses below are conducted at room temperature.

### X-ray powder diffraction studies

X-ray powder diffraction patterns were collected on a PANalytical Empyrean X-ray diffractometer of the Netherlands equipped with a transmission-reflection sample stage with an automated 3*15 zero background sample holder. The radiation source used was (Cu, kα, Kα1(Å):1.540598; Kα2(Å):1.544426; Kα2/Kα1 intensity ratio: 0.50), wherein the voltage was set at 45KV and the current was set at 40 mA. The beam divergence of the X-rays, *i.e.* the effective size of the X-ray confinement on the sample, was 6.6 mm. The continuous scanning mode of θ-θ was adopted, and an effective 2θ range of 3° to 40° was obtained. A proper amount of sample was placed at the position of the circular groove of the zero-background sample rack under the environmental condition (about 18°C to 32 °C), the sample was lightly pressed with a clean glass slide to obtain a flat plane, and the zero-background sample rack was fixed. The samples were scanned at a scan step of 0.013 ° in the range of 3° to 40 ° 2θ ± 0.2 ° to obtain a traditional X-ray powder diffraction pattern. The software used for Data collection was a Data Collector, and Data was analyzed and presented by using Data Viewer and HighScore Plus.

### High performance liquid chromatography (HPLC) detection of crystalline forms

The instrument used for liquid chromatography analysis is Agilent HPLC 1260 series, which is used for the analysis of solid-state stability of samples.

### DESCRIPTION OF THE DRAWINGS

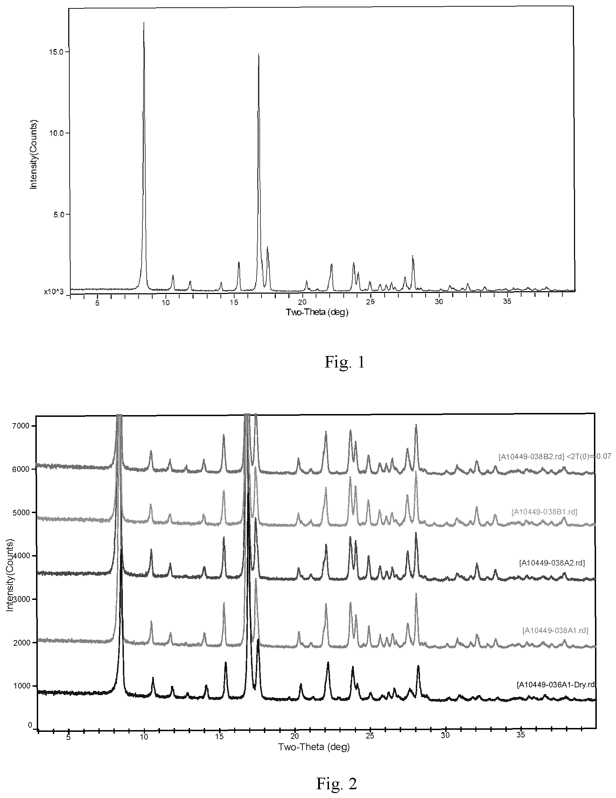

Figure 1 shows an X-ray powder diffraction pattern of crystalline form III prepared in the examples of the present invention
Figure 2 shows an X-ray powder diffraction pattern of crystalline form III prepared in the examples of the present invention stored in various conditions

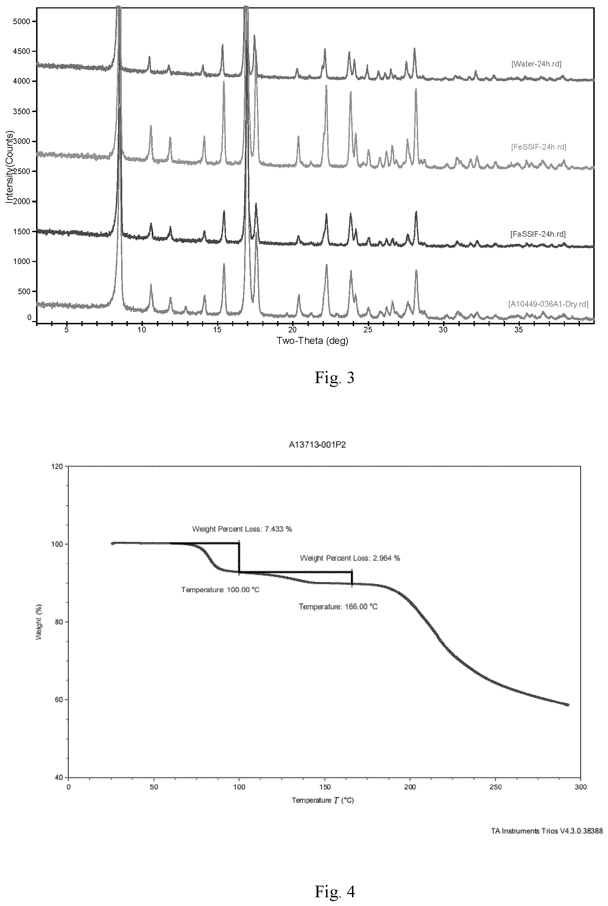

Figure 3 shows an X-ray powder diffraction pattern of crystalline form III prepared in the examples of the present invention dissolved in various solvents
Figure 4 shows a differential scanning calorimetry (DSC) curve of crystalline form III prepared in the examples of the present invention

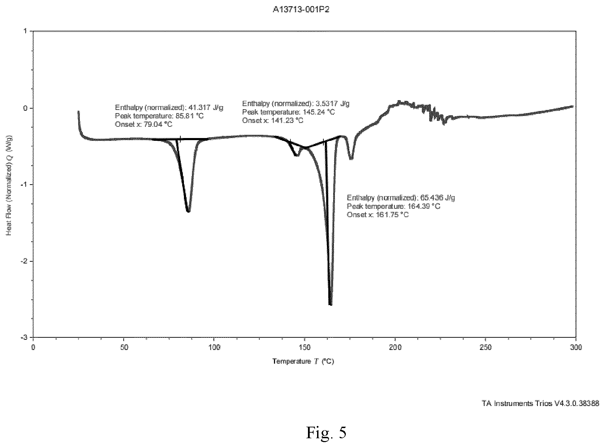

Figure 5 shows a thermogravimetric analysis curve (TGA) of crystalline form III prepared in the examples of the present invention

### DETAILED DESCRIPTION

In order to make those skilled in the art better understand the technical solutions of the present invention, non-limiting embodiments will be further disclosed to further describe the present invention.

The reagents used in the invention are either commercially available or can be prepared by the methods described herein.

In the present invention, µM means micromoles per liter; mmol means millimole; equiv means an equivalent; aw means water activity.

### Example 1. Synthesis of (3aR,4R,6R,6aR)-4-(4-aminopyrrolo[2,1-f][1,2,4] triazin-7-yl)-6-(hydroxymethyl-2,2-dimethyltetrahydrofuro[3,4-d][1,3]dioxole-4-carbonitrile (compound 5)

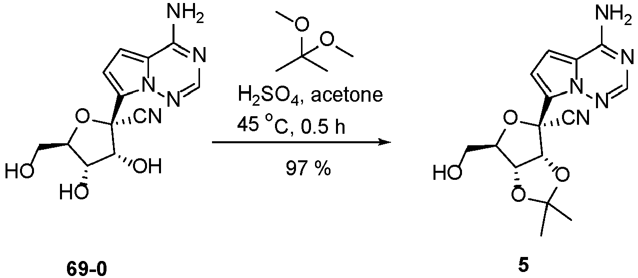

5.62 g of Compound 69-0 was dissolved in 30 mL of acetone, 11.50 mL of 2, 2-dimethoxypropane and 1.34 mL of 98% sulfuric acid were added, which was stirred at 45 °C for half an hour and cooled to room temperature, and the organic solvent was removed by rotary evaporation. The mixture was extracted with 100 mL of ethyl acetate and 100 mL of saturated sodium bicarbonate solution three times, the ethyl acetate layers were combined, dried over anhydrous sodium sulfate, and filtered to remove the sodium sulfate. The organic solvent was removed by rotary evaporation and purified by column chromatography (eluent: petroleum ether/ethyl acetate (v/v) = 1/2) to obtain 6.20 g of compound 5 (white solid, 97% yield). The obtained compound 5 was tested for hydrogen spectrum, and the results were as follows:
Hydrogen spectrum: ¹H NMR (400 MHz, Chloroform-*d*) δ 7.95 (s, 1H), 7.11 (d, *J=* 4.7 Hz, 1H), 6.69 (dd, *J* = 4.8, 2.4 Hz, 1H), 5.77 (s, 2H), 5.42 (d, *J* = 6.6 Hz, 1H), 5.24 (dd, *J* = 6.6, 2.4 Hz, 1H), 4.67 (q, *J=* 1.9 Hz, 1H), 3.99 (dd, *J=* 12.5, 1.9 Hz, 1H), 3.84 (dd, *J=* 12.5, 1.7 Hz, 1H), 1.81 (s, 3H), 1.40 (s, 3H).

### Example 2. Synthesis of ((3aR,4R,6R,6aR)-6-(4-aminopyrrolo[2,1-f][1,2,4] triazin-7-yl)-6-cyano-2,2-dimethyltetrahydrofuro[3,4-d][1,3] dioxol-4-yl) methyl isobutyrate (compound INT-1)

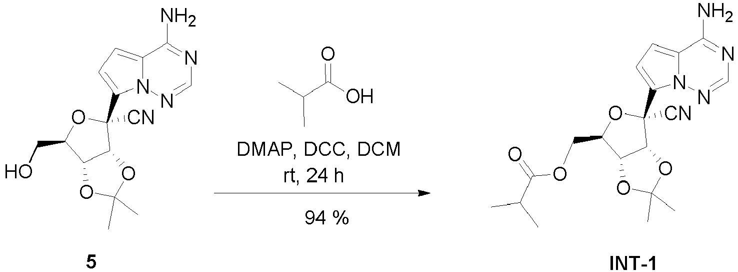

1.50 g of Compound 5 was dissolved in 15 mL of dichloromethane, and 0.42 mL of isobutyric acid and 55.40 mg of 4-dimethylaminopyridine were added thereto, and after stirring for 10 min, 1.02 g of dicyclohexylcarbodiimide was added, and the mixture was stirred at room temperature for 24 hours. The mixture was purified by column chromatography (eluent: petroleum ether/ethyl acetate (v/v) = 1/1) to obtain 1.71 g of compound INT-1 (white solid, 94% yield). The obtained compound INT-1 was tested for hydrogen spectrum, and the results were as follows:
Hydrogen spectrum:¹H NMR (400 MHz, CDCl₃, ZQF-RD01-2) δ (ppm): 7.99 (s, 1H), 6.99 (d, *J*=4.6 Hz, 1H), 6.62 (d, *J*=4.6 Hz, 1H), 5.72 (br, 2H), 5.49 (d, *J*=6.8 Hz, 1H), 4.93-4.90 (dd, *J*=6.8 Hz, 4.3 Hz, 1H), 4.61-4.58 (q, *J*=4.4 Hz, 1H), 4.44-4.26 (m, 2H), 2.61-2.50 (m, 1H), 1.77 (s, 3H), 1.42 (s, 3H), 1.17-1.14 (q, *J*=3.8 Hz, 6H).

Carbon spectrum: ¹³C NMR (100 MHz, CDCl₃, ZQF-RD01-2) δ (ppm): 176.7, 155.2, 147.3, 123.5, 117.2, 116.7, 115.6, 112.6, 100.0, 83.8, 83.0, 82.0, 81.4, 63.1, 33.8, 26.4, 25.6, 18.9.

### Example 3. Synthesis of ((2R,3S,4R,5R)-5-(4-aminopyrrolo[2,1-f][1,2,4] triazin-7-yl)-5-cyano-3,4-dihydroxytetrahydrofuran-2-yl) methyl isobutyrate(compound A)

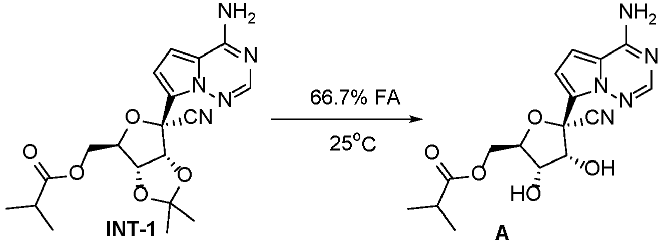

Water (310 mL) was added into a reaction vessel at the temperature of 20-30 °C, formic acid (620 mL) was added , and then INT-1 (1.0eq,93g) was added, the mixture was stirred at room temperature for more than 24 hours, sample was took and the reaction endpoint was detected (if the peak area of INT-1 is less than 0.5%, it is considered as the reaction has ended), the mixture was cooled to-5.0 °C, ethyl acetate (186 mL) was added, 50% sodium carbonate aqueous solution was added to adjust pH to 7-8, the mixture was stirred for more than 1 hour, filtered, and the filter cake was washed with water (288 mL x 3) to obtain the compound A.

### Example 4: characterization detection of crystalline form

The compound prepared in Example 1 was subjected to X-ray powder diffraction detection, differential scanning calorimetry detection, and thermogravimetric analysis detection. The results were shown in Figures 1 to 3, respectively. A novel crystalline form of compound A was obtained, named crystal form III.

### Example 5: Dynamic water vapor adsorption detection of crystalline form III

The dynamic water vapor adsorption test results showed that crystalline form III does not have hygroscopicity. Under the conditions of 80% RH and 25 °C, the weight gain was less than 0.2%, and the crystalline form did not change after testing, proving that the crystalline form stability of crystalline form III is good.

### Example 6: Grinding experiment of crystalline form III

Crystalline form III was ground manually with a mortar and pestle for 5 minutes, and XRPD of the ground solid was tested. After grinding for five minutes, the crystalline structure did not change, indicating that the physical stability of crystalline form III is good.

### Example 7: Solid state stability test

A certain amount of crystalline form III of compound A as a sample was placed under conditions of 40 °C/75% RH (open) and 60 °C (closed) for one week, and XRPD and HPLC of the sample were tested. The results were shown in the table below. Crystalline form III was physically and chemically stable under two acceleration conditions for 14 days.

**Table 1 Solid State Stability Test Results**

| **Sample** | **Initial purity (Peak area%)** | **Purity-1 week (Peak area%)** | | **Crystal-1 week** | |
|---|---|---|---|---|---|
| | | **60 °C & closed** | **40 °C /75% RH, open** | **60 °C & closed** | **40 °C /75% RH, open** |
| Sample 1 | 99.71% | 99.71% | 99.68% | No change | No change |
| Sample 2 | | 99.65% | 99.71% | No change | No change |

### Example 8: Results of a solubility test

The results of a solubility test of crystalline form III of compound Ain biological media (SGF, FaSSIF, and FeSSIF) and water at 37 ° C were shown in the table below. Crystalline form III has the highest solubility in SGF (>7 mg/mL). The solubility in pure water, FeSSIF, and FaSSIF were all greater than 1 mg/mL. After the solubility test, there was no significant change in solution pH.

**Table 2: Results of solubility test**

| **Solvent** | **Solubility (mg/mL)** | | | **XRPD (24 h)** | **pH (24 h)** |
|---|---|---|---|---|---|
| | **0.5 h** | **2 h** | **24 h** | | |
| SGF (pH=1.20) | >7* | | | N/A | 1.37 |
| FeSSIF (pH=4.97) | 1.63 | 1.66 | 1.68 | Crystalline form III | 4.96 |
| FaSSIF (pH=6.53) | 1.22 | 1.26 | 1.19 | Crystalline form III | 6.50 |
| Water (pH=6.67) | 1.11 | 1.22 | 1.24 | Crystalline form III | 6.43 |

| | | | | | |
|---|---|---|---|---|---|
| *Visual observation | | | | | |

The method of the present invention has been described through the preferred embodiments, and it is obviously, a skilled artisan can make any alterations, changes or combinations thereof appropriately to implement and apply the present invention without departing from the content, spirit and scope of the present invention. The skilled in the art can learn from the present invention and improve the process parameters appropriately. It should be noted that it can be readily apparent to those of ordinary skill in the art that certain modifications may be made thereto within the scope of the invention.

## Claims

1. A crystalline form of the compound of formula A, 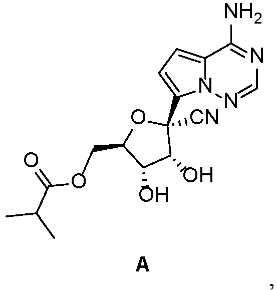 which is **characterized by** an X-ray powder diffraction pattern of crystalline form III having characteristic peaks at 2θ values of 8.47 ± 0.2°, 16.87 ± 0.2°, 17.47 ± 0.2°, 22.13 ± 0.2° and 28.66 ± 0.2°.

2. The crystalline form of claim 1, wherein the X-ray powder diffraction pattern of crystalline form III has characteristic peaks at 2θ values of 8.47±0.2°, 16.87±0.2°, 17.47±0.2°, 22.13±0.2°, 28.66±0.2°, 10.52±0.2°, 11.80±0.2°, 14.04±0.2°.

3. The crystalline form of claim 1, wherein the X-ray powder diffraction pattern of crystalline form III has characteristic peaks at 2θ ± 0.2° values of 8.475, 10.523, 11.808, 14.043, 15.331, 16.878, 17.470, 20.293, 21.106, 22.131, 23.733, 24.074, 24.545, 24.915, 25.688, 26.147, 26.528, 26.778, 27.526, 28.079, 28.669, 30.126, 30.783, 31.044, 31.689, 32.110, 32.793, 33.304, 34.868, 35.431 35.746 , 36.494, 37.007, 37.887.

4. The crystalline form of claim 1, wherein the X-ray powder diffraction pattern of crystalline form III is substantially as depicted in Fig.1.

5. A process for preparing crystalline form III of any one of claims 1 to 4, comprising: adding water, organic acid and INT-1 into a reaction vessel at the temperature of 20 °C to 30 °C; stirring for more than 24 hours at room temperature; cooling to -5.0 °C to 5.0 °C after the reaction is finished; adding an esters solvent; adding an alkaline aqueous solution to adjust pH value to 7-8; stirring for more than 1 hour; filtering; washing the filter cake with water; drying;

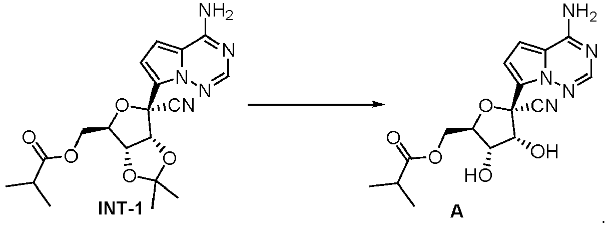

6. The process of claim 5, wherein the organic acid is formic acid or acetic acid, and the volume ratio of the organic acid to water is 3:1 to 1: 1.

7. The process of claim 5, wherein the esters solvent is selected from one or more of methyl formate, ethyl acetate or isobutyl acetate.

8. A composition comprising the crystalline form of any one of claims 1 to 4 and a pharmaceutically acceptable adjuvant.

9. Use of the crystalline form of any one of claims 1 to 4 or the composition of claim 8 for the manufacture of a product for preventing, alleviating and/or treating a coronavirus infection, or the replication or propagation of a homologous variant thereof, and the cytopathic effects thereof.

10. The use of claim 9, wherein the coronavirus comprises MHV-A59, HCoV-229E, HCoV-OC43, HCoV-NL63, HCoV-HKU1, SARS-CoV, MERS-CoV, SARS-CoV-2, mouse hepatitis virus, Feline infectious peritonitis virus, canine coronavirus, bovine coronavirus, avian infectious bronchitis virus or porcine coronavirus.
